Europäisches Patentamt

European Patent Office

Office européen des brevets

(11) Veröffentlichungsnummer: **0 208 955**
A2

## EUROPÄISCHE PATENTANMELDUNG

(21) Anmeldenummer: 86108552.0

(22) Anmeldetag: 23.06.86

(51) Int. Cl.⁴: **G 01 L 19/00**, G 01 L 7/08, A 61 B 5/02

(30) Priorität: 17.07.85 DE 3525536

(71) Anmelder: **Peter von Berg, Extrakorporale Systeme -Medizintechnik GmbH, Benzstrasse 2, D-8011 Kirchheim (DE)**

(43) Veröffentlichungstag der Anmeldung: 21.01.87 **Patentblatt 87/4**

(72) Erfinder: **von Berg, Peter, Schwabener Weg 1, D-8011 Neukeferloh (DE)**

(84) Benannte Vertragsstaaten: **AT BE CH DE FR GB IT LI LU NL SE**

(74) Vertreter: **EGLI-EUROPEAN PATENT ATTORNEYS, Widenmayerstrasse 5, D-8000 München 22 (DE)**

(54) Verfahren zur Herstellung eines druckübertragenden Kontaktes zwischen der Membran eines Druckdomes und dem Druckübertrager eines Messwandlers und Druckmessvorrichtung zur Ermittlung des physiologischen Druckes von Fluiden des menschlichen oder tierischen Körpers.

(57) Bei einem Verfahren zur Herstellung eines druckübertragenden Kontaktes zwischen der Membran (17) eines Druckdomes (1) und dem Druckübertrager (22) eines Meßwandlers (2) einer Druckmeßvorrichtung zur Ermittlung des physiologischen Druckes von Fluiden des menschlichen oder tierischen Körpers wird von einer Einrichtung zur Fixierung des Druckdomes (1) und des Meßwandlers (2) in der druckübertragenden Kontaktstellung der Membran (17) und des Druckübertragers (22) Gebrauch gemacht, welche ein Zusammenführen der Membran (17) und des Druckübertragers (22) in deren druckübertragende Kontakt- Endstellung ohne gegenseitige Rotationsbewegungen ermöglicht.

Verfahren zur Herstellung eines druckübertragenden Kontaktes zwischen der Membran eines Druckdomes und dem Druckübertrager eines Meßwandlers und Druckmeß- vorrichtung zur Ermittlung des physiologischen Druckes von Fluiden des menschlichen oder tierischen Körpers

Die Erfindung bezieht sich auf ein Verfahren zur Herstellung eines druckübertragenden Kontaktes zwischen der Membran eines Druckdomes und dem Druckübertrager eines Meßwandlers einer Druckmeßvorrichtung zur Ermittlung des physiologischen Druckes von Fluiden des menschlichen oder tierischen Körpers, bei welchem die Membran und der Druckübertrager bis zu ihrer Endlage ihres druckübertragenden gegenseitigen Kontaktes aufeinander zubewegt werden.

Die Erfindung bezieht sich auch auf eine Druckmeßvorrichtung zur Ermittlung des physiologischen Druckes von Fluiden des menschlichen oder tierischen Körpers mit einem Druckdom, der fluidisch mit dem Körper verbindbar ist und eine druckauslenkbare Membran aufweist. Die Druckmeßvorrichtung weist ferner einen Druckübertrager, der in druckübertragenden Kontakt mit der Membran bringbar ist und eine Fixiereinrichtung zur Fixierung der Membran und des Druckübertragers in ihrer druckübertragenden Kontakt-Endstellung auf.

Unter Fluiden des menschlichen oder tierischen Körpers werden Körperflüssigkeiten oder -gase, insbesondere Blut verstanden.

Ein derartiges Verfahren und eine derartige Druckmeßvorrichtung sind bereits in der US-Patentschrift 4 185 641 bzw. der zugehörigen deutschen Offenlegungsschrift 29 30 869 beschrieben. Die bekannte Druckmeßvorrichtung besteht im wesentlichen aus zwei Teilen, nämlich einem Druckdom und einem Meßwandler. Der Druckdom besteht im wesentlichen aus einem Gehäuse, das unter Aussparung einer Seite einen Innenraum umgibt. Die ausgesparte Seite ist von einer druckauslenkbaren Membran abgedeckt. Es ist somit im Druckdom ein hermetisch gegenüber dem Außenraum abgeschlossener Innenraum geschaffen. Dieser ist über mindestens eine Fluidleitung an den Körper einer zu untersuchenden Person so anschließbar, daß durch diese Leitung jene Körperflüssigkeit oder jenes Körpergas, dessen Druck gemessen werden soll, in den Druckdom eingelassen wird. In der Regel befinden sich noch weitere Anschlüsse an der Fluidleitung, etwa zum Einleiten eines koagulationshemmenden Mittels im Falle der sogenannten "blutigen Blutdruckmessung".

Ein solcher Druckdom wird nach der keimfreien Herstellung in einer sterilen Verpackung ausgeliefert und vom Verbraucher dieser entnommen. Hierbei wird eine Infizierung des Körperfluides ausgeschlossen, da der Innenraum steril und hermetisch gegenüber der Umgebung abgeschlossen ist. Nach der Benutzung wird der Druckdom weggeworfen.

Der Meßwandler der Druckmeßanordnung weist einen Druckübertrager, meistens in Form einer ebenfalls druckauslenkbaren Membran auf, die einen Meßfühler bildet. Dieser Druckübertrager begrenzt eine Seite eines ihn halternden Gehäuses. Die Meßeinrichtung im Inneren des Gehäuses spricht auf die Auslenkung dieser Membran an und erzeugt ein für den Druck repräsentatives Messignal.

Der Druckdom und der Meßumwandler werden so aneinander befestigt, daß deren Membranen bewegungsübertragend aneinander anliegen. Um diese gegenseitige Anlage noch zu fördern, kann vor dem Zusammensetzen der beiden Einrichtungen noch eine der Membranen mit einer Kontaktflüssigkeit benetzt werden, die aufgrund ihrer Viskosität jeden sich etwa bildenden Zwischenraum überbrückt.

Es ist bekannt, Druckdom und Meßwandler mit einem Vater-bzw. Muttergewinde zu versehen und zusammenzuschrauben. Werden diese beiden Teile nur soweit zusammengeschraubt, daß deren Membranen gerade aufeinander satt aufsitzen, dann wird die den Meßwert bildende Auslenkung der Druck-dommembrane verfälschungsfrei auf die Druckübertrager-membrane übertragen. Die beiden Membranen weisen nämlich in diesem Fall keine den Meßwert verfälschende Verformung auf. Auch wenn ein Fremdkörper beim Zusammenschrauben der beiden Gehäuse zwischen die Membrane geriete, würde dieser Fremdkörper nur verhältnismäßig geringen Schaden anrichten, da er nur mit einer geringen Andruckkraft zwischen den beiden Membranen eingebettet wäre.

In der Praxis hat sich aber herausgestellt, daß die obenbeschriebene ideale Membran-Positionierung nicht praktizierbar ist. Vielmehr werden in der Regel die beiden Gehäuse zu fest miteinander verschraubt, um sicher zu sein, daß sich die beiden Membranen ausreichend berühren. In einem solchen Fall liegen aber die beiden Membranen bereits dann schon mit einer bestimmten Vorspannung aneinander an, wenn im Innenraum der Druckdose noch kein Druck vorliegt. Diese Vorspannung erzeugt bereits ein Messignal. Es ist somit vor jeder Inbetriebnahme der Meßanordnung ein Nullpunktausgleich erforderlich.

Ferner können die beiden Membranen aneinander haften, wenn sie bereits mit einer gewissen Vorspannung aneinander liegen, so daß sie bei einem weiteren Verschrauben einer relativen Verdrehung, und somit einer Torsion ausgesetzt werden. Diese Torsion bringt ihrerseits Spannung in die Membranen, welche deren Auslenkverhalten bei Druckeinwirkung verfälschen. Neben dem Verschieben des Nullpunkts, das an sich ausgeglichen werden kann, wird hierdurch auch noch die Messcharakteristik verändert. Ein Ausgleich der Meßcharakteristik ist aber kaum möglich.

Gelangt außerdem vor dem zu festen Verschrauben der beiden Gehäuse ein Fremdkörper zwischen die Membranen, kann eine Beschädigung oder gar ein Durchreißen einer oder beider Membranen die Folge sein. Wird beispielsweise die Druckdommembran beschädigt und somit durchlässig, dann kann dieser Schaden nicht ohne weiteres festgestellt werden. Die Meßwandlermembran liegt nämlich dichtend gegen die Druckdommembran an und dichtet deren Riß ab. Es kann somit durch diesen Riß hindurch eine unbemerkte Infizierung des zu messenden Körperfluids von Seiten der Meßwandlermembran zustandekommen.

Um die vorgenannten Probleme der Nacheichung zu mildern, weist die gattungsbildende, bekannte Meßanordnung (DE-OS 29 30 869/ US-PS 4 185 641)) einen Bajonettverschluß auf, der federnd nachgiebige Teile enthält, mittels welcher die beiden Membranen gegeneinander gedrückt werden. Hierbei sorgen die federnd nachgiebigen Teile dafür, daß stets eine annähernd gleiche Vorspannung die beiden Membrane aufeinander drückt, so daß eine Reproduzierbarkeit auch des Nullpunktes möglich ist und die Nullpunkteinstellung zwischen zwei Meßvorgängen vereinfacht werden oder gar unterbleiben kann.

Die obenbeschriebenen Probleme, die bei der Gewindeverbindung zwischen den beiden Gehäusen nur dann auftreten, wenn man diese zu fest zusammenschraubt, treten nun aber zwangsläufig bei der bekannten gattungsbildenden Anordnung ständig auf, weil dort das lose Aufeinanderliegen der beiden Membranen nicht möglich ist, denn diese werden stets durch die Federkraft der nachgiebigen Teile des Bajonettverschlusses zusammengedrückt. Bei der Betätigung des Bajonettverschlusses werden gleichzeitig auch die beiden Membranen gegeneinander verdreht, so daß die die Meßcharakteristik verfälschende Torsion der Membranen stets und deren Beschädigung dann auftreten kann, wenn zwischen die beiden Membranen ein scharfkantiger Fremdkörper gelangt.

Ausgehend von dieser Problemlage strebt die Erfindung den Erfolg an, beim einleitend genannten Verfahren zur Herstellung eines druckübertragenden Kontaktes zwischen der Membran eines Druckdomes und dem Druckübertrager eines Meßwandlers einer Druckmeßvorrichtung zur Ermittlung des physiologischen Druckes von Fluiden des menschlichen oder tierischen Körpers sowie bei der einleitend genannten Druckmeßvorrichtung eine gute Reproduzierbarkeit der Meßcharakteristik zu erzielen. Dies wird dadurch erreicht, daß bei dem genannten Verfahren zur Herstellung des druckübertragenden Kontaktes zwischen der Membran des Druckdomes und dem Druckübertrager des Meßwandlers die Membran und der Druckübertrager frei von einer gegenseitigen relativen Rotationsbewegung aufeinander zubewegt werden, bis sie ihre Endlage ihres druckübertragenden gegenseitigen Kontaktes eingenommen haben.

In vorrichtungsmäßiger Hinsicht wird der angestrebte Erfolg dadurch erreicht, daß die Fixiereinrichtung der eingangs genannten Druckmeßvorrichtung ein rotationsfreies Aufeinanderzubewegen der Membran des Druckdomes und des Druckübertragers des Meßwandlers bis zu deren druckübertragender Kontakt-Endstellung ermöglicht.

Nach der Erfindung müssen die beiden aneinander zu befestigenden Gehäuse, d.h. der Druckdom und der den Druckübertrager halternde Gehäuseteil, nicht mehr - wie bisher - zumn Zwecke ihrer Befestigung dann relativ zueinander verdreht werden, wenn sich die Membran des Druckdomes und der Druckübertrager des Meßwandlers bereits gegenseitig in Angriff befinden. Vielmehr sollen nach der Erfindung die beiden Gehäuseteile zumindest in der Endphase ihrer gegenseitigen Befestigung geradlinig, bevorzugt in Richtung der Flächennormalen der Membran/ des Druckübertragers gegeneinander gedrückt werden. Hierdurch wird die die Meßcharakteristik verändernde Torsionsbeanspruchung der Membran/des Druckübertragers vermieden. Gleichzeitig wird die Gefahr von Membranbeschädigungen durch scharfkantige Partikel, die sich unter Druck längs der Membranoberfläche bewegen, praktisch ausgeschlossen.

Gemäß einem bevorzugten Ausführungsbeispiel weist die Fixiereinrichtung der Druckmeßvorrichtung wenigstens eine, vorzugsweise zwei, den Druckdom und ein den Druckübertrager, der vorzugsweise ebenfalls als druckauslenkbare, elastische Membran ausgebildet ist, halterndes Gehäuseteil überbrückende Klammer auf. Die Klammern können lose mit dem Druckdom mitgeliefert werden. Diese Ausführungsform hat den Vorteil, einer besonders einfachen und wirtschaftlichen Ausgestaltung der Fixiereinrichtung.

Es ist beispielsweise auch möglich, die beiden Gehäuseteile einseitig, zum Beispiel unter Bildung eines Scharnieres, miteinander zu verhaken, dann zusammenzuklappen und in dieser Stellung schließlich durch eine dem Scharnier gegenüberliegende Klammer zu befestigen. Vorzugsweise ist aber eines der Gehäuse mit zwei einander diametral gegenüberliegenden Klammern ausgebildet, so daß die beiden Gehäuse völlig geradlinig zusammengesteckt oder aufeinander gelegt und dann in ihrer Endlage durch Umlegen der Klammern und durch deren Eingriff in eine Gegenausbildung, vorzugsweise eine Gehäuseausbildung, aneinander befestigt werden können.

Die Klammern können, wie bereits erwähnt, an einem der beiden Gehäuse angebracht sein. Grundsätzlich ist es auch möglich, an beiden Gehäusen jeweils Klammern anzuordnen. Bevorzugt sind jedoch die Klammern am Gehäuse des Druckdomes angebracht, also an jenem Teil, das den einleitend genannten Wegwerfteil bildet. Die Anlenkungsstellen der Klammern sind nämlich verhältnismäßig schwer sauber zu halten, so daß zur Vermeidung hygienischer Probleme die Klammern nach jeder Benutzung zusammen mit dem Druckdom weggeworfen werden können.

Um die beiden Gehäuse und somit deren Membranen bequem und sicher genau aufeinander ausrichten zu können, ist gemäß einer Weiterbildung des Grundgedankens der Erfindung mindestens an einem der beiden Gehäuse mindestens eine Führung vorgesehen, zu der passend am anderen Gehäuse mindestens eine Gegenführung angeordnet ist. Die Führungen bzw. Gegenführungen erstrecken sich parallel zur Flächennormalen der jeweiligen Membranebene. Sie ermöglichen somit eine geradlinige Annäherung der beiden Gehäuse und somit der beiden Membranen, so daß die beiden Membranen in ihrer druckübertragenden Kontakt-Endstellung genau aufeinander ausgerichtet bzw. zentriert sind.

Die Führungen können beispielsweise als Axialnuten in einer am zugehörigen Gehäuse angeformten Muffe ausgebildet sein, wobei als Gegenführungen komplementäre Warzen an der Stirnseite des anderen Gehäuses an geordnet sein können. Gemäß einer bevorzugten Ausgestaltung der Erfindung sind pro Klammer zwei Führungsleisten angeordnet, welche die Klammer oder einen Teil davon beiderseits führen. Der Klammer gegenüberliegend ist am gegenüberliegenden Gehäuse eine Nase ausgebildet bzw. angeformt. Diese Nase erfüllt somit gleichzeitig die Funktion der Gegenführung als auch jener Gehäuseausbildung, welche von der Klammer zum Zusammenhalten der beiden Gehäuse umgriffen wird. Die Führungsleisten können mit ihren von der jeweiligen Klammer abgewandten Oberfläche durch eine Umfangsleiste verbunden sein, welche insgesamt eine rohrmuffenartige Verlängerung des Gehäuses bildet, die lediglich von den axial verlaufenden Schlitzen zwischen den Führungsleisten unterbrochen ist.

Gemäß der bereits oben erörterten bevorzugten Ausführungsform ist das die Klammern tragende Gehäuse das Druckdomgehäuse, welches nun gemäß der vorliegenden Weiterbildung auch die rohrmuffenartige Verlängerung trägt. Deren Innenkanten bilden nämlich Schmutzsammelstellen, so daß alle schwer zu reinigenden Ausbildungen und Elemente der gesamten Meßanordnung vorteilhafterweise bei jenem Teil vorliegen, welches als Wegwerfteil ausgebildet ist, während das andere Teil, nämlich der Drucküberträger, lediglich warzenartig überstehende Nasen aufweist und im übrigen ein glattes Gehäuse aufweisen kann.

Die beiden, beiderseits einer jeden Klammer angeordneten Führungsleisten weisen gemäß einer weiteren Ausgestaltung der Erfindung Lagerbohrungen bzw. -vertiefungen auf, in welche Achsstummel einrastbar sind, die am einen Ende der Klammer, und zwar zu beiden Seiten, an die Klammer angeformt sind. Hierbei bilden die Führungsleisten infolge ihrer Steifigkeit eine zuverlässige Lagerung für die Klammer.

Gemäß einer bevorzugten Ausgestaltung der Erfindung weist die Klammer einen etwa F-förmigen Querschnitt auf, wobei an den beiden Seiten des freien Ende des oberen Querschenkels des F die oben genannten Achsstummel angeformt oder angebracht sind. Der untere Querschenkel des F ist so angeordnet, daß er mit seiner Oberfläche genau die ihm zugewandte Rückseite der zugehörigen Nase in Schliesstellung untergreift und satt gegen diese anliegt. Hierbei befindet sich der Auflagepunkt praktisch genau unterhalb der Schwenkachse der Klammer, so daß die aus dem Zusammendrücken der beiden Membramen resultierende Kräfte die
Klammer nicht öffnen können. Bevorzugt ist der dem freien
Ende zugewandte Abschnitt der oberen Fläche des unteren
Querschenkels des F abgeschrägt, und erleichtert hierdurch ein Untergreifen der Nase.

Ferner ist die Klammer bevorzugt so ausgebildet, daß ihr
nach außen weisender Rücken in Schließstellung bündig den
Zwischenraum zwischen den beiden Führungsleisten abschließt, so daß eine glatte Außenoberfläche gebildet
ist.

Bei der obenbeschriebenen Ausgestaltung der Erfindung ist
die Klammer unmittelbar am zugehörigen Gehäuse schwenkbar
angebracht; gemäß einer bevorzugten Ausgestaltung der
Erfingung ist es aber auch möglich, die Klammer selbst an
einem Zwischenhebel schwenkbar anzubringen, welcher dann
seinerseits schwenkbar mit dem Gehäuse verbunden ist.
Hierbei kann die Anbringung der Klammer zusammen mit dem
Zwischenhebel ebenso innerhalb zweier Führungsleisten
erfolgen wie dies bei der oben beschriebenen, unmittelbar
am Gehäuse angelenkten Klammer der Fall ist.

Die Verwendung eines Zwischenhebels hat den Vorteil, daß
das Halteende der Klammer beim Umgreifen der Gehäuseausbildung, insbesondere der oben genannten Nase, nicht
kurzzeitig eine Kraft ausübt, welche die beiden Membrane
zusammenzudrücken trachtet, sondern es ist bei abstehenden Zwischenhebeln möglich, die Endkralle der Klammer
frei um die Gehäuseausbildung, zum Beispiel der Gehäuseboden oder die obenbeschriebene Nase, herumzuführen und
dann durch Umlegen des Zwischenhebels bzw. durch Andrücken des von der Kralle abgewandten Endes der Klammer

0208955

die Kralle geradlinig gegen das dazugehörige Gehäuse heranzuziehen. Hierbei liegt in Schließstellung die vom Gehäuse abgewandte Fläche des Zwischenhebels an die dem Gehäuse zugewandte Fläche der Klammer an oder wird von dieser umgeben.

Es ist grundsätzlich auch möglich, die Klammer mit der Nase bzw. Gehäuseausbildung zu verrasten oder an dieser zu befestigen und hiermit eine zuverlässige Halterung zu bewirken. Gemäß einer bevorzugten Ausgestaltung der Erfindung ist aber die Klammer an jenem Gehäuse in ihre Schließstellung arretierbar, an welchem sie auch schwenkbar angebracht ist. Die Arretierung trägt somit nicht dazu bei, die beiden Gehäuse mit einer bestimmten Kraft zusammenzuziehen, sondern verhindert lediglich die Bewegung der Kralle.

Gemäß einer bevorzugten Ausgestaltung der Erfindung weist die Klammer an ihren beiden Längsseiten jeweils Längsrippen auf, die in Schließstellung der Klammer in Gegenrillen einrastbar sind, die in den der Klammer zugewandten Seiten der Führungsleisten ausgebildet sind. Hierdurch wird die Klammer - und ggf. mit dieser auch der Zwischenhebel - zuverlässig in seiner Schließlage gehalten.

Gemäß einer weiteren Ausgestaltung der Erfindung sind die freien Enden der Klammer über die Führungsleisten hinaus verlängert und als Handhabe ausgebildet, wobei aus der Stellung der Handhabe unmittelbar ersichtlich ist, ob die zugehörige Klammer geschlossen ist oder nicht.

Der Gegenstand der Erfindung wird anhand der beigefügten schematischen Zeichnung beispielsweise noch näher erläutert. In dieser Zeichnung zeigt:

Fig. 1    ein Ausführungsbeispiel des Druckdomes und des Meßwandlers der Druckmeßanordnung    vor dem Zusammensetzen,

Fig. 2    nach dem Zusammensetzen,

Fig. 3    eine Schnittlängslinie III - III in Fig. 2,

Fig. 4    eine Einzelheit des Schnitts der Fig. 3, in vergrößerter Darstellung und im Schrägbild,

Fig. 5    eine Darstellung ähnlich Fig. 2, jedoch mit modifiziertem Druckdom,

Fig. 6    eine Ausführungsform mit einer Klammer mit Zwischenhebel.

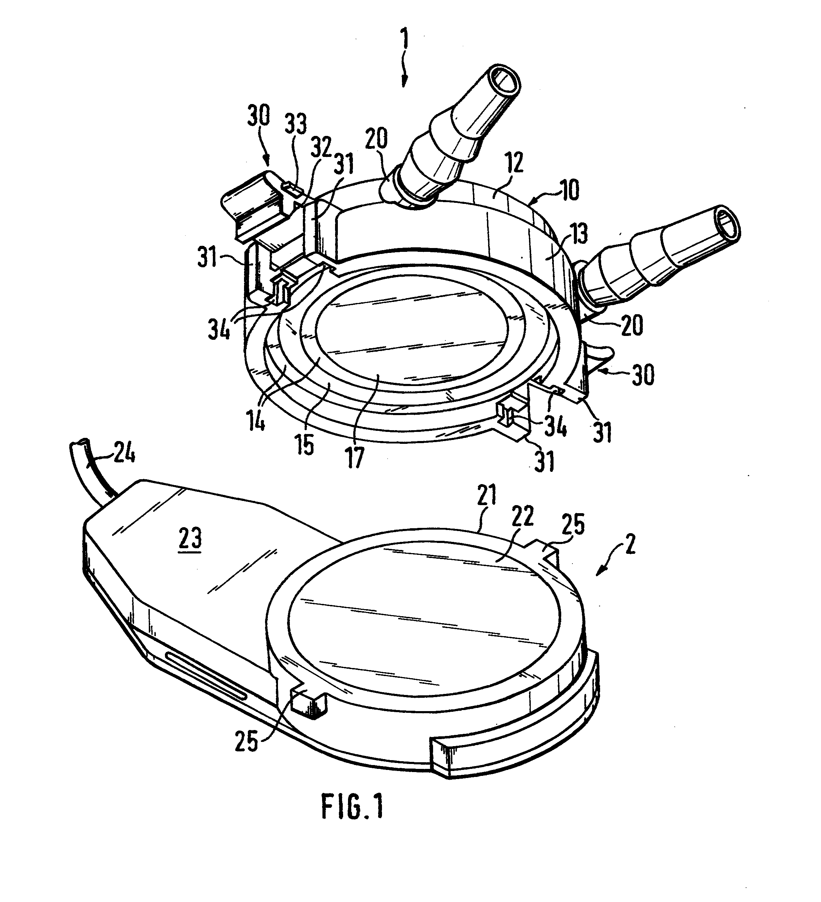

Fig. 1 zeigt einen  Druckdom 1 und einen Meßwandler 2 in einer Stellung kurz vor deren Zusammensetzen.

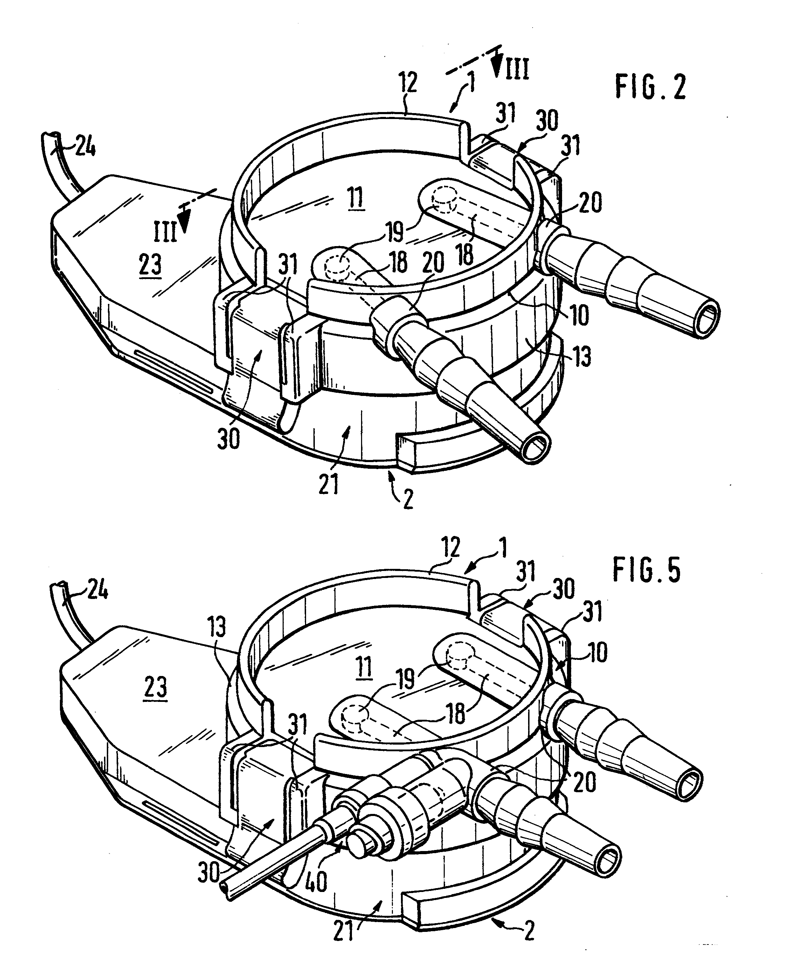

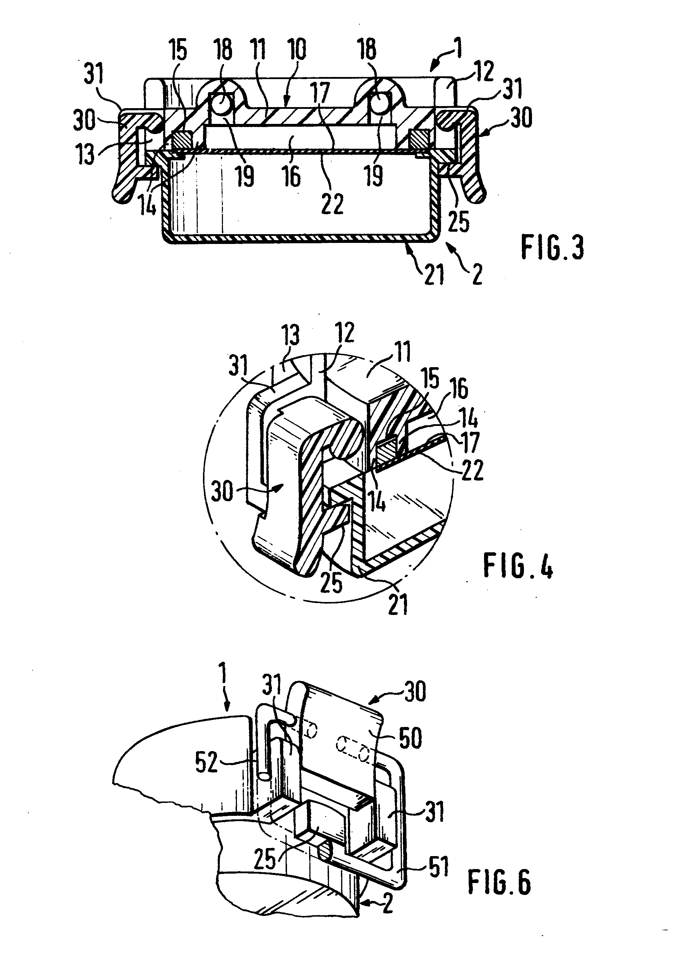

Der Druckdom 1 weist ein Gehäuse 10 mit einem kreisförmigen Gehäuseboden 11 (s. Fig. 2 und 3) auf.  Entlang der Peripherie der einen Gehäusebodenseite ist eine erste Ringmuffe 12 und dieser - auf der anderen Gehäusebodenseite gegenüberliegend - eine ringförmige Umfangswand 14 angeformt, deren Außendurchmesser kleiner als jener der Ringmuffe 12 ist. In der Umfangswand 14 ist eine Ringnut 15 eingebracht, die von deren in Fig. 1 unteren Ringrand ausgeht und der ersten Ringnut 12 zugewandt ist. Am radial äusseren Rand der Umfangswand 14 ist eine zweite Ringmuffe 13 angebracht. Diese weist in entgegengesetzte Richtung wie die erste Ringmuffe 12 und hat einen größeren Durchmesser als jene.

Die Umfangswand 14 umgibt den Innenraum 16 des Druckdoms 1. Dieser ist auf seiner einen Seite vom Gehäuseboden 11 und auf seiner anderen Seite von einer druckauslenkbaren Membran 17 hermetisch abgeschlossen. Die Membran 17 ist mit bekannten Haltemitteln längs ihres Außenumfanges in der Ringnut 15 befestigt.

In dem Gehäuseboden 11 sind zwei Leitungen 18 eingeformt, die sich parallel zu ihm erstrecken und aufeinander zulaufen. Sie können aber auch zueinander parallel verlaufen. An ihrem inneren Ende treffen sie auf einen zum Innenraum 16 hin führenden Verbindungskanal 19.

An ihren äußeren Enden münden sie in Rohrstutzen 20, welche die erste Ringmuffe 12 nach außen durchdringen. Wie aus Fig. 1 und 5 ersichtlich, sind in die Rohrstutzen 20 Schlauchverbindungen für eine Schlauchleitung eingesetzt.

Jede Ringmuffe 12 und 13 weist an einander diametral gegenüberliegenden Stellen radial bis in Höhe der Ringwand 14 schlitzartig verlaufende Aussparungen auf, deren Breite einer noch später zu beschreibenden Klammer 30 entspricht. Die Aussparungen beider Ringmuffen 12, 13 fluchten mit der Klammer 30 in deren Schließstellung. Beiderseits der schlitzartigen Aussparungen in der zweiten Ringmuffe 13 ist eine radial nach außen vorstehende Führungsleiste 31 für die Klammer 30 angeformt. Die Höhe dieser Führungsleisten 31 ist so bemessen, daß sie die ihr zugeordnete Klammer 30 in deren Schließstellung seitlich abdecken (Fig. 2 und 3).

Die Klammer 30 (siehe auch Fig. 4) hat einen im wesentlichen F-förmigen Querschnitt. Das freie Ende des oberen Querschenkels dieses F-Querschnitts ist zu einem kreisförmigen Querschnitt verdickt. An den beiden Enden dieses verdickten Bereiches sind - in der Zeichnung nicht sichtbare - Achsstummel angeordnet, die in zugeordneten Aussparungen in den Führungsleisten 31 drehbar gelagert sind. Beim Einführen einer Klammer 30 bzw. deren Achsstummel zwischen die beiden Führungsleisten 31 werden letztere leicht auseinandergedrückt, bis die Achsstummel in die zugehörigen Aussparungen einfallen können.

Der zweite, d.h. untere Querschenkel des F-förmigen Querschnitts bildet an seiner Oberseite eine Stützfläche 32, die in ihrem vorderen Bereich zum freien Ende des Querschenkels hin leicht abfällt.

Das untere Ende des Längsschenkels des F-Querschnitts ist von den Querschenkeln leicht weggeschweift, steht in Schließlage über die beiden Führungsleisten 31 hinaus und ist als Handhabe ausgebildet. Im unteren Abschnitt des Längsschenkels ist an dessen beiden Längsseiten, den beiden Führungsleisten 31 aber noch gegenüberliegend, jeweils eine vorstehende Längsrippe 33 ausgebildet (Fig. 1). Diese Längsrippen 33 fallen in Schließstellung in ihnen jeweils gegenüberliegende Längsrillen 34 in den Führungsleisten 31 ein.

Der in Fig. 1 gezeigte Meßwandler 2 weist einen im wesentlichen zylinderförmigen Gehäuseteil 21 mit einer ebenen Endfläche auf. Diese setzt sich aus einer verformbaren Membran 22 und einem die Membran 22 umgebenden Ring zusammen. Die Membran 22 dient als unmittelbarer Drucküberträger, welcher den von der Membran 17 ausgeübten Druck unmittelbar an die nachgeschalteten Einheiten des Meßwandlers überträgt.

Der Gehäuseteil 21 geht an seinem der Membran 22 abgewandten Ende seitlich in einen weiteren Gehäuseteil 23 über, der etwa die Form eines flachen Kastens hat. Dessen vom zylindrischen Gehäuseteil 21 entferntes Ende verjüngt sich nach außen. Von dessen freier Endfläche geht ein elektrisches Leitungskabel 24 aus, das die Druckwerte in Form elektrischer Signale weiterleitet.

Im Inneren des Meßwandlers 2 ist ein Meßumformer angeordnet, der die Auslenkung der Membran 22 in ein entsprechendes elektrisches Signal umwandelt, das über das Kabel 24 einer Meßeinrichtung zugeführt wird.

Der Außendurchmesser des zylindrischen Gehäuseteils 21 und der Innendurchmesser der zweiten Ringmuffe 13 sind so aufeinander abgestimmt, daß die beiden Teile 21 und 13 mit leichtem Spiel ineinandergeschoben werden können, bis der Ring der Endfläche des Gehäuseteils 21 auf der diesem zugewandten ringförmigen Endfläche der Umfangswand 14 des Druckdomes 1 aufsitzt. In dieser Stellung liegen die beiden Membranen 17 und 22 satt aneinander.

Am Außenumfang des Ringes des zylindrischen Gehäuseteils 21 sind diametral einander gegenüberliegend zwei auswärtsweisende Nasen 25 angeformt, die in Radial- und Umfangsrichtung geschnitten jeweils rechteckig sind. Deren dem Druckdom 1 zugewandte Fläche geht in radialer Richtung bündig in die Endfläche des Ringes des Gehäuseteils 21 über.

Der Druckdom 1 und der Meßwandler 2 werden vor ihrem Zusammensetzen so angeordnet, daß deren Membranen 17 und 22 einander parallel gegenüberliegen. Dann werden die beiden Teile 1 und 2 so gegeneinander verdreht, daß die Nasen 25 genau den Zwischenräumen zwischen den beiden Führungsleistenpaaren 31 gegenüberliegen. Anschließend werden der Druckdom 1 und der Meßwandler 2 ineinander geschoben, bis die dem Druckdom 1 zugewandten Flächen der Nasen 25 satt auf dem zwischen den Führungsleisten 31 liegenden Teil der zweiten Ringmuffe 13 aufsitzen. Dann werden die Klammern 30, die vor dem Zusammenführen von Druckdom 1 und Meßwandler 2 die in Fig. 1 gezeigte Lage eingenommen hatten, in welcher sie seitwärts abragen, nach unten in die aus Fig. 2, 3 und 4 ersichtliche Lage geschwenkt. Hierbei untergreift, wie besonders aus Fig. 4 ersichtlich, die Stützfläche 32 des unteren Querschenkels des F-förmigen Querschnitts genau die von der Druckdose 1 abgewandte Fläche der zugehörigen Nase 25 und liegt beständig gegen diese an. Die vordere Abschrägung der Stützfläche 32 (Fig. 1 und 4) stellt sicher, daß die Klammer 30 um die zugehörige Nase 25 bequem herumgeschwenkt werden kann.

Wenn die Klammer 30 ganz umgelegt ist, rasten ihre seitlichen Längsrippen 33 in die komplementären Längsrillen 34 in den Führungsleisten 31 ein. Hierdurch werden die Klammern 30 fest in ihrer Lage gehalten. Hierbei stimmt die aus Längsschenkel und oberem Querschenkel gebildete Kontur der Klammer 30 mit jener der benachbarten Führungsleisten 31 im wesentlichen überein, mit Ausnahme des leicht auswärts geschweiften, verlängerten unteren Endes des Längsschenkels; dieses Ende dient als bequeme Handhabe zum Öffnen der Klammer 30 und ist ohne weiteres ergreifbar.

All jene Teile des Druckdomes 1, die mit dem Körperfluid, vorzugsweise Blut, in Berührung kommen, bestehen aus einem physiologisch unbedenklichen Kunststoff; abgesehen von der Membran, ist dieser möglichst unnachgiebig. Als Material für die Membran kommt beispielsweise Silicon-Gummi infrage. Das Gehäuse 10 des Druckdomes 1 ist bevorzugt im Druckguß hergestellt. Die Klammern 30 sind aus einem harten Kunststoff gebildet, können aber beispielsweise auch aus Metall gebildet sein, wobei sie bevorzugt durch Abtrennen von extrodiertem Stangenmaterial hergestellt sind, das besonders maßhaltig ist.

Das Gehäuse 21 des Meßwandlers 2 ist aus möglichst widerstandsfähigem Kunststoff oder Metall hergestellt. Das Material der Membran 22 braucht physiologisch nicht unbedenkblich zu sein, da es nicht mit einem Körperfluid in Berührung gelangt.

In Fig. 5 ist eine andere Ausführungsform eines Druckdomes 1 gezeigt: Einer der beiden Rohrstutzen 20 ist nach außen hin verlängert und weist außerhalb der erste Ringmuffe 12 eine Spüleinrichtung 40 zur dosierten Einführung eines Mittels auf, das der Koagulation von Blut entgegenwirkt. Die Spüleinrichtung ist in bekannter Weise auch für eine Stoßspülung ausgelegt. Trotz dieser Zusatzeinrichtung wird die gegenseitige Befestigung von Druckdom 1 und Meßwandler 2 mittels der Klammern 30 in keiner Weise beeinträchtigt.

In Fig. 6 ist ein weiteres Ausführungsbeispiel der Erfindung gezeigt, das weitgehend mit den vorgenannten Ausführungsbeispielen übereinstimmt. Insbesondere die Führungsleisten 31, die Nase 25 und die ein Schwenklager bildenden, in den einander zugewandten Oberflächen der Führungsleisten 31 ausgebildeten Aufnahmebohrungen für die Achsstummel der Klammer 30 sind gleichgeblieben; die Klammer 30 wurde jedoch modifiziert.

Sie weist einen Schließhebel 50 in Form eines flachen Quaders auf, der, ebenso wie die Klammer 30 der vorbeschriebenen Ausführungsbeispiele, mit seitlich ausgebildeten oder angeformten Zapfen oder Achsstummeln in den entsprechenden Aufnahmebohrungen in den beiden Führungsleisten 31 schwenkbar gelagert ist. Der Schließhebel 50 erstreckt sich jedoch in seiner Schließstellung nicht zur Nase 25 hin, sondern von dieser weg, und zwar wiederum über die Führungsleisten 31 hinaus. An seinem oberen (freien) Ende ist er leicht vom Druckdom 1 weggeschwungen ausgebildet, so daß er bequem ergriffen und in eine Stellung geschwenkt werden kann, in welcher er sich vom Druckdom 1 nach außen abspreizt.

In jenem Teil des Schließhebels 50, der in Schließstellung über die beiden Führungsleisten 31 übersteht, ist seitlich jeweils eine Aufnahmebohrung ausgebildet, in welcher die beiden Endschenkel eines im wesentlichen rechteckigen Rahmenbügels 51 schwenkbar eingesetzt sind. In Schließstellung umgreift der Rahmenbügel 51 von außen her die beiden Führungsleisten 31 und untergreift die Nase 25. Er ist vorzugsweise in seinem mit der Nase 25 in Anlage tretenden Bereich abgeflacht ausgebildet.

Die die Führungsleisten 31 von der Seite her umgreifenden Schenkel des Rahmenbügels 51 können mit den ihnen zugewandten Flächen der Führungsleisten 31 verrastet sein und hierbei in Längsnuten 52 liegen, die parallel zu den Führungsleisten 31 angeordnet sind. Ein Vorteil dieser Ausbildung liegt darin, daß man sich durch einen Blick auf die Klammeranordnung davon überzeugen kann, ob tatsächlich ein ordnungsgemäßer Eingriff zwischen dem Rahmenbügel 51 und der Nase 25 hergestellt ist, weil dieser Eingriffsbereich ohne weiteres von außen erkennbar ist.

Der Rahmenbügel 51 kann aus Draht gebogen sein, ist jedoch bevorzugt aus einem geeigneten Kunststoff geformt.

0208955

Verfahren zur Herstellung eines druckübertragenden
Kontaktes zwischen der Membran eines Druckdomes und
dem Druckübertrager eines Meßwandlers und Druckmeßvorrichtung zur Ermittlung des physiologischen
Druckes von Fluiden des menschlichen oder
tierischen Körpers

A N S P R Ü C H E

1. Verfahren zur Herstellung eines druckübertragenden
Kontaktes zwischen der Membran (17) eines Druckdomes
(1) und dem Druckübertrager (22) eines Meßwandlers
(2) einer Meßvorrichtung zur Ermittlung des physiologischen Druckes von Fluiden des menschlichen oder
tierischen Körpers, bei welchem die Membran (17) und der Druckübertrager (22) bis zu ihrer Endlage ihres druckübertragenden gegenseitigen Kontaktes aufeinander zubewegt werden,

dadurch g e k e n n z e i c h n e t , daß die Membran (17) und der Druckübertrager (22) frei von einer gegenseitigen relativen Rotationsbewegung aufeinander zubewegt werden.

2. Verfahren nach Anspruch 1, dadurch gekennzeichnet, daß die Membran (17) und/oder der Druckübertrager (22) vor ihrer gegenseitigen Kontaktierung mit einer Kontaktflüssigkeit benetzt werden.

3. Druckmeßvorrichtung zu Ermittlung des physiologischen Druckes von Fluiden des menschlichen oder tierischen Körpers mit

a) einem Druckdom (1) der fluidisch mit dem Körper verbindbar ist und eine druckauslenkbare Membran (17) aufweist,

b) einem Druckübertrager (22), der in druckübertragenden Kontakt mit der Membran (17) bringbar ist und

c) einer Fixiereinrichtung (25, 30) zur Fixierung der Membran (17) und des Druckübertragers (22) in ihrer druckübertragenden Kontakt-Endstellung,

dadurch g e k e n n z e i c h n e t , daß

d) die Fixiereinrichtung (25, 30) ein rotationsfreies Aufeinanderzubewegen von Membran (17) und Druckübertrager (22) bis zu deren druckübertragender Kontakt-Endstellung ermöglicht.

4. Druckmeßvorrichtung nach Anspruch 3, dadurch gekennzeichnet, daß die Fixiereinrichtung (25, 30) wenigstens eine den Druckdom (1) und ein den Drucküberträger (22) halterndes Gehäuseteil (21) überbrückende Klammer (30) aufweist.

5. Druckmeßvorrichtung nach Anspruch 4, dadurch gekennzeichnet, daß an der Außenseite des einen Gehäuses (10) (von Druckdose (1) oder Drucküberträger (22)) zwei einander diametral gegenüberliegende, schwenkbare Klammern (30) angebracht sind, welche durch Verschwenken mit einer Gehäuseausbildung (25) des anderen Gehäuses (21) in Halteeingriff bringbar sind, sobald sich die Membran (17) und der Drucküberträger (22) in ihrer druckübertragenden Kontaktstellung befinden.

6. Druckmeßvorrichtung nach einem der Ansprüche 3 bis 5, dadurch gekennzeichnet, daß der Drucküberträger (22) ebenfalls eine druckauslenkbare Membran ist.

7. Druckmeßvorrichtung nach einem der Ansprüche 3 bis 6, dadurch gekennzeichnet, daß an den Gehäusen (10, 22) von Druckdose und/oder Drucküberträger (22) Führungen (31) angeordnet sind, die senkrecht zur/zum zugehörigen Membran/Drucküberträger (17, 22) angeordnet sind, und daß am jeder Führung (31) gegenüberliegenden Gehäuse (10, 21) eine mit dieser Führung (31) in Führungseingriff bringbare Gegenführung (25) angeordnet ist.

8. Druckmeßanordnung nach Anspruch 7, dadurch gekennzeichnet, daß die Führung (31) zwei die Klammer (30) beiderseits führende Führungsleisten (31) aufweist und die Gehäuseausbildung als Gegenführung ausgebildet ist und hierzu eine der Klammer (30) gegenüberliegende Nase (35) aufweist, deren Breite
kleiner als der Abstand zwischen den beiden Führungsleisten (31) ist.

9. Druckmeßanordnung nach Anspruch 7 oder 8, dadurch
gekennzeichnet, daß am einen Ende der Klammer (30)
bzw. eines Klammerteils (50) beiderseits dieser ein
überstehender Achsstummel ausgebildet ist, der
jeweils in eine Aufnahmevertiefung in der benachbarten Führungsleiste (31) drehbar aufgenommen ist.

10. Druckmeßanordnung nach Anspruch 9, dadurch gekennzeichnet, daß

    a)    die Klammer (30) einen etwa F-förmigen Querschnitt aufweist,

    b)    die Achsstummel am freien Ende des oberen Querschenkels der F-förmigen Klammer (30) angeordnet sind,

    c)    der untere Querschenkel etwa die gleiche Länge
        wie der obere Querschenkel aufweist,

    d)    die Unterseite der Nase (25) eben ausgebildet
        und zum zugehörigen Druckübertrager (22) parallel angeordnet ist und

    e)    die Klammer (30) so angeordnet ist, daß ihr
        unterer Schenkel in Schließstellung satt gegen
        die Unterseite der Nase (25) anliegt.

11. Druckmeßanordnung nach Anspruch 9, dadurch gekennzeichnet, daß an der Oberseite des unteren Querschenkels im Bereich dessen freien Endes eine zu
diesem hin abfallende Abschrägung ausgebildet ist.

12. Druckmeßanordnung nach einem der Ansprüche 5 bis 11, dadurch gekennzeichnet, daß die Klammer (30) im Bereich ihres von der Gehäuseausbildung (25) abgewandten Endes schwenkbar am Ende eines Zwischenhebels angebracht ist, dessen anderes Ende schwenkbar am Druckdom (1) oder Gehäuseteil (21) für den Drucküberträger (22) angebracht ist, wobei in Schließstellung der Zwischenhebel im wesentlichen zwischen dem Druckdom (1) bzw. Gehäuseteil (21) und der Klammer (30) angeordnet ist.

13. Druckmeßanordnung nach einem der Ansprüche 5 bis 12, dadurch gekennzeichnet, daß die Klammer (30) an jenem Gehäuse/Gehäuseteil (10/21) in ihrer Schließstellung arretierbar ist, an welchem sie schwenkbar angebracht ist.

14. Druckmeßanordnung nach Anspruch 8 und 13, dadurch gekennzeichnet, daß die Klammer (30) beiderseits überstehende Längsrippen (33) aufweist, die in Schließstellung in komplementäre Längsrillen (34) einrastbar sind, die in den einander zugewandten Flächen der Führungsleisten (31) ausgebildet sind.

15. Druckmeßanordnung nach einem der Ansprüche 8 bis 14, dadurch gekennzeichnet, daß das freie Ende der Klammer (30) über die Führungsleisten (31) übersteht und als Handhabe ausgebildet ist.

16. Druckmeßanordnung nach Anspruch 8, dadurch gekennzeichnet, daß die Klammer (30) einen schwenkbar in den Führungsleisten (31) gelagerten Schließhebel (50) und eine schwenkbar an diesem gelagerten, mit der Nase (25) in Eingriff bringbaren Schwenkhebel (51) aufweist.

17. Druckmeßanordnung nach Anspruch 16, dadurch gekennzeichnet, daß der Schwenkhebel als Rahmenbügel (51) ausgebildet ist, der in Schließstellung des Schließhebels (50) die beiden Führungsleisten (31) umgreift.

18. Druckmeßanordnung nach Anspruch 17, dadurch gekennzeichnet, daß der Rahmenbügel (51) im Bereich der Auflage der Nase (25) eine an deren Eingriffsfläche angepaßte Abflachung aufweist.

19. Druckmeßanordnung nach Anspruch 17 oder 18, dadurch gekennzeichnet, daß der Rahmenbügel in Schließstellung in eine zu beiden Seiten der Führungsleisten (51) geführte Längsnut (52) einrastet.

FIG.1

FIG. 2

FIG. 5

0208955

3/3

FIG.3

FIG.4

FIG.6